# EUROPÄISCHE PATENTSCHRIFT

(11) **EP 1 169 428 B1**
(45) Veröffentlichungstag und Bekanntmachung des Hinweises auf die Patenterteilung: **23.03.2005**
(21) Anmeldenummer: 00926833.5
(22) Anmeldetag: 06.04.2000
(51) Int. Cl.: C12M 1/00

(54) **PHOTOBIOREAKTOR MIT VERBESSERTEM LICHTEINTRAG DURCH OBERFLÄCHENVERGRÖSSERUNG, WELLENLÄNGENSCHIEBER ODER LICHTTRANSPORT**
PHOTOBIOREACTOR WITH IMPROVED SUPPLY OF LIGHT BY SURFACE ENLARGEMENT, WAVELENGTH SHIFTER BARS OR LIGHT TRANSPORT
PHOTOBIOREACTEUR A INCIDENCE AMELIOREE DE LA LUMIERE PAR AUGMENTATION DE LA SURFACE, ELEMENTS DE DECALAGE DE LA LONGUEUR D'ONDES OU TRANSPORT DE LA LUMIERE

(30) Priorität: 13.04.1999 DE 19916597
(43) Veröffentlichungstag der Anmeldung: 09.01.2002
(73) Patentinhaber: Fraunhofer-Gesellschaft zur Förderung der angewandten Forschung e.V., 80686 München (DE)
(72) Erfinder: TRÖSCH, Walter, D-70329 Stuttgart (DE); SCHMID-STAIGER, Ulrike, D-72654 Neckartenzlingen (DE); ZASTROW, Armin, D-79102 Freiburg (DE); RETZE, Alexander, D-79114 Freiburg (DE); BRUCKER, Franz, D-79102 Freiburg (DE)
(74) Vertreter: Albrecht, Thomas, Dr.
(86) Internationale Anmeldenummer: PCT/EP2000/003089
(87) Internationale Veröffentlichungsnummer: WO 2000/061719

(56) Entgegenhaltungen:
- WO-A-91/08314
- DE-A- 4 440 081
- DE-C- 19 611 855
- US-A- 3 959 923
- US-A- 4 473 970
- US-A- 4 952 511
- US-A- 5 162 051

## Beschreibung

Die Erfindung betrifft einen neuen Photobioreaktor zur Produktion von Biomasse.

Photobioreaktoren sind Fermenter, in denen phototrophe Mikroorganismen, wie Algen, Cyanobakterien und Purpurbakterien kultiviert werden, in denen also entweder das Wachstum und die Vermehrung dieser Zellen ermöglicht wird oder die Produktion unterschiedlicher Substanzen mittels phototropher Zellen gefördert wird.

Solche Photobioreaktoren sind beispielsweise in den folgenden Druckschriften beschrieben:
(i) "Biomass and Icosapentaenoic Acid Productivities from an Outdoor Batch Culture of *Phaeodactylum tricornutum* UTEX 640 in an Airlift Tubular Photobioreactor", Appl. Microbiol. Biotechnol. (1995), 42, S. 658-663,
(ii) "Autotrophic Growth and Carotenoid Production of *Haematococcus pluvialis* in a 30 Liter Air-Lift Photobioreactor", Journal of Fermentation and Bioengineering (1996), Bd. 82, Nr. 2, S. 113-118,
(iii) "light Energy Supply in Plate-Type and Light Diffusing Optical Fiber Bioreactors", Journal of Applied Phycology (1995), 7, S. 145 - 149,
(iv) "A Simplified Monodimensional Approach for Modeling Coupling between Radiant Light Transfer and Growth Kinetics in Photobioreactors", Chemical Engineering Science (1995) , Bd. 50, Nr. 9, S. 1489-1500.

Außerdem beschreibt die Druckschrift DE 44 40 081 A1 ein Verfahren und eine Vorrichtung zur Erhöhung der Produktivität in Biokollektoren für phototrophe Mikroorganismen, wobei durch Einziehen einer porösen Schicht die Oberfläche für die Besiedelung durch Biokatalysatoren vergrößert wird. Dabei wird die Anströmung der besiedelten Flächen dadurch gewährleistet, dass das aufwärtsströmende Kultursubstrat durch die poröse Schicht hindurchgeführt wird. Durch die Ausrichtung der Porenkanäle im Sonnenlicht kann eine optimale Ausnutzung von schräg auf den Reaktor fallendem Licht gewährleistet werden. Das Verfahren eignet sich für die Herstellung von Ausscheidungsprodukten phototropher Mikroorganismen und Mikroalgen.

Das US-Patent 4 952 511 beschreibt einen Photobioreaktor für die Kultivierung photosynthetischer Mikroorganismen. Dieser Photobioreaktor umfasst einen Tank, mindestens eine Lichtkammer, die sich in den Tank hinein erstreckt, und mindestens eine Lampe hoher Intensität, deren Licht in die Lichtkammern geleitet wird. Jede der Lichtkammern besitzt mindestens eine transparente Wand und Vorrichtung zur im wesentlichen gleichförmigen Verteilung des Lichts der Lampe über die transparente Wand.

Das US-Patent 3 959 923 beschreibt schließlich eine Vorrichtung zur Kultivierung von Algen, bei der Wasser zusammen mit den Algen und Nährstoffen entlang mäandrierender Strömungskanäle abwechselnd durch breite, dunkle und enge, beleuchtete Zonen strömt.

Ein Haupteinsatzgebiet von Photobioreaktoren ist die Erzeugung von Mikroalgen, die einen Anteil von 30% der weltweit erzeugten Primärproduktion an Biomasse haben. Dabei sind sie die wichtigsten CO2-Konsumenten. Mikroalgen sind deshalb geeignet, umweltentlastend zu wirken, wenn man sie zur regenerativen Stoffproduktion nutzt. Stoffe, die so produziert werden, tragen dann zur Reduzierung der CO₂-Emission in die Atmosphäre bei, da sie fossil hergestellte Stoffe substituieren.

Zu den Mikroalgen zählen einerseits die prokaryotischen Cyanobakterien als auch eukaryotische mikroskopische Algenklassen. Diese Organismen liefern eine Vielfalt von Substanzklassen, die sich für Zwecke der Pharmazie, Kosmetik, Nahrung, Tierernährung sowie für technische Zwecke (z.B. Schwermetall-Adsorption) einsetzen lassen. Wichtige Stoffklassen sind hierbei lipophile Verbindungen wie z.B. Fettsäuren, Lipide, Sterole und Carotinoide, hydrophile Stoffe wie Polysaccharide, Proteine bzw. Aminosäuren und Phycobiliproteine (Pigmente) sowie die Gesamtbiomasse als proteinreicher, nukleinsäurearmer Rohstoff.

In der BRD wie auch im internationalen Maßstab verstärkt sich der Trend zur Ablösung von synthetischen Wirkstoffen durch den Einsatz von Naturstoffen mit gleichwertigen bzw. verbesserten Applikationseigenschaften. Von zunehmendem Interesse sind antioxidative Wirkstoffkomplexe und mehrfach ungesättigte Fettsäuren mit therapeutischer Potenz im Bereich der Kosmetik, der Medizin und im Gesundheitsmarkt (health food market). Zu diesen interessanten Antioxidantien zählen die Tocopherole (Vitamin E) und Carotinoide wie β-Carotin und Astaxanthin.

Die Wirtschaftlichkeit der durch Mikroalgen produzierten Stoffe wird zunächst durch die Produktivität der ausgesuchten Algenspezies bestimmt. Jedoch nur, wenn gleichzeitig ein hoher Umwandlungswirkungsgrad von solarer Strahlungsenergie in die gewünschte Biomasseform erreicht wird und der energetische Aufwand und die Kosten für Herstellung, Installation und Betrieb der Anlage äußerst gering gehalten werden. Hohe Biomasseproduktivität ist ein Problem der optimalen Lichtverteilung pro Volumen. Die Absorption von Licht durch Algen führt zu starker Lichtabnahme mit zunehmender Schichtdicke, gleichzeitig findet eine gegenseitige Selbstbeschattung statt. Dieses Phänomen führt zu einer theoretischen Schichtbildung im Reaktor:
(1) äußere Algenschicht, die zu hohen Lichtintensitäten ausgesetzt ist, die zu Photoinhibition führen kann,
(2) mittlere Schicht mit idealer Beleuchtung,
(3) innere Algenschicht mit Lichtmangel und hoher Respirationsrate.

Der Erfindung liegt daher die Aufgabe zugrunde, einen Photobioreaktor bereitzustellen, bei dem die zur Verfügung stehende Solarstrahlung so eingekoppelt wird und verteilt werden kann, daß alle Mikroorganismen ortsunabhängig eine gleichhohe Photosyntheseaktivität aufweisen.

Gegenstand der Erfindung ist ein Photobioreaktor, welcher einen Reaktorraum aufweist, der eine Oberflächenvergrößerung größer als die geradflächige umhüllende Fläche eines Volumens besitzt.

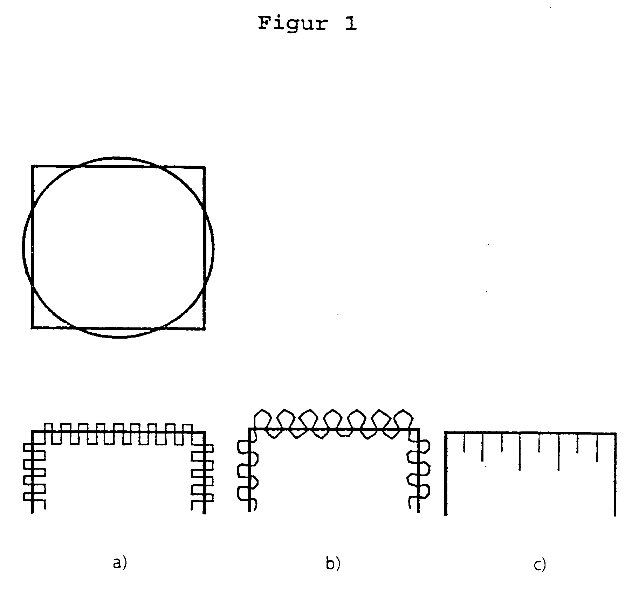

Diese Oberflächenvergrößerung führt zu einer besseren räumlichen Verteilung des Lichts über den Reaktorquerschnitt und damit zu einer Optimierung der Lichtintensität im gesamten Reaktor im Vergleich zu aus dem Stand der Technik bekannten Photobioreaktoren. Deren Reaktorraum besteht üblicherweise aus Rohren oder sogenannten "Tubes". Deren Querschnitt ist kreisförmig. Daneben sind Photobioreaktoren mit Reaktorräumen bekannt, deren Querschnitt rechteckig ist. Ein solcher Querschnitt hat mehr Fläche als Volumenumhüllende als der Kreisquerschnitt eines Rohres. Dies ist in Figur 1 schematisch dargestellt.

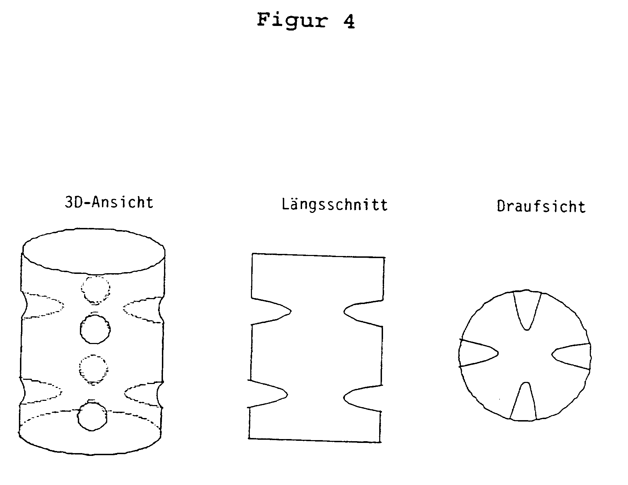

Der erfindungsgemäße Photobioreaktor weist eine gegenüber den vorgenannten bekannten Reaktorgeometrien eine Oberflächenvergrößerung auf, wie sie in Figur 1 beispielhaft dargestellt ist. Hierin bezeichnet (a) eine mäanderförmige und (b) eine sinusförmige Reaktoroberfläche. (c) bezeichnet eine Reaktorgeometrie mit lichtdurchlässigen Stegen. Eine weitere Ausführungsform ist in Figur 4 dargestellt, wobei Glasfortsätze nach innen die Oberflächenvergrößerung darstellen.

All diesen Geometrien ist gemein, daß die Reaktoroberfläche gegenüber bekannten Geometrien vergrößert ist.

Bei dem erfindungsgemäßen Photobioreaktor sind prinzipiell sämtliche Reaktorraumgeometrien verwendbar, die eine gegenüber der geradflächig Umhüllenden (Quadrat oder Rechteck im Querschnitt) eine Oberflächenvergrößerung aufweisen.

Der Reaktorraum des erfindungsgemäßen Photobioreaktors besteht aus einem lichtdurchlässigen Material, vorzugsweise aus Glas oder Plexiglas.

Gemäß einer weiteren Ausführungsform der Erfindung wird die oberflächenvergrößernde Geometrie des Reaktorraums durch ein Glasrohr realisiert, in dessen Innenraum Glasfortsätze ragen. Diese Glasfortsätze sind auf der Innenoberfläche alternierend senkrecht und schräg angebracht.

Gleichzeitig wird durch die Glasfortsätze die Turbulenz in der Flüssigphase erhöht. Anstelle von Glas kann auch ein anderes lichtdurchlässiges Material, wie z.B. Plexiglas, verwendet werden.

Durch die Erhöhung der Turbulenz wird der sogenannte "Flashing-Light-Effekt" erzielt. Der Flashing-Light-Effekt besagt, daß für eine maximale Photosyntheseaktivität ein hohe Lichtintensität in kurzen Abständen (> 1 Hz) ausreichend ist. Dies kann durch eine turbulente Strömungsführung im Reaktor erzielt werden, wodurch die Zellen in kurzen Abständen an der Reaktoroberfläche hohen Lichtintensitäten ausgesetzt sind, und deshalb in den nachfolgenden Dunkelphasen die gesammelte Lichtenergie verarbeiten können.

Die Erfindung betrifft daher weiterhin einen Photobioreaktor, dessen Reaktorraum neben einer erhöhten Oberfläche Einrichtungen für eine turbulente Strömungsführung aufweist.

Wie bereits erwähnt, kann diese Turbulenz durch eine oberflächenvergrößernde Geometrie des Reaktorraums, insbesondere durch Glasfortsätze, die sich auf der Reaktorrauminnenwand befinden, erreicht werden. Eine turbulente Strömungsführung kann auch weiterhin durch den Einbau von statischen Mischern (Strombrechern) erzielt werden. Diese Einbauten können außerdem - wie die vorgenannten Glasfortsätze - zusätzlich Licht in den Reaktor leiten. Eine weitere Möglichkeit zur Erzeugung von Turbulenzen in dem erfindungsgemäßen Photobioreaktor besteht darin, eine Begasungsvorrichtung vorzusehen, die bei entsprechender Begasungsrate den gewünschten Effekt erzielt. Auch durch das Vorsehen von strömungsführenden Einbauten kann der Flashing-Light-Effekt verbessert werden, indem definierte Frequenzen für die Beleuchtungszeit eingestellt werden.

Das starke Mischen mit möglichst viel Turbulenz führt zu einer Lichtverteilung, indem die Algen zum Licht gebracht werden. Dadurch kann die Häufigkeit und die Dauer der "Beleuchtungsphasen" definiert gesteuert werden.

Die Energiedichte in dem Reaktorraum des erfindungsgemäßen Photobioreaktors kann weiterhin durch die Verwendung sogenannter Wellenlängenschieber erhöht werden. Durch den Wellenlängenschieber wird der durch phototrophe Mikroorganismen nicht absorbierbare Anteil des Lichts so konvertiert, daß ein möglichst großer Lichtanteil oder die Gesamtheit der Strahlung in jenes Frequenzband verschoben werden kann, das von dem Photozentrum des eingesetzten phototrophen Mikroorganismus absorbierbar ist. Somit wird die holometrische Strahlungsdichte spezifisch so erhöht, daß pro Reaktorvolumen gegenüber mit Normallicht bestrahlten Bioreaktoren die Produktivität wesentlich erhöht wird.

Dabei kann der Wellenlängenschieber zwischen einem Reflektor und dem eigentlichen Reaktorraum angeordnet sein. Der Wellenlängenschieber kann aber auch zwischen der Lichtquelle und dem Reaktorraum vorgesehen werden, so daß auf den Reflektor verzichtet werden kann.

Ein Verzicht auf den Reflektor ist ebenfalls möglich, wenn der Wellenlängenschieber in Form von Stäben, Platten, Fasern oder Partikeln in dem Reaktorraum vorliegt. Des weiteren kann der Wellenlängenschieber auch als Anstrich direkt auf der Reaktorwand an der Außenseite bzw. Innenseite des Reaktorraums vorliegen.

Substanzen, die in der Lage sind, die Wellenlänge zu verschieben, sind dem Fachmann an sich bekannt und beispielsweise in den folgenden Druckschriften beschrieben:
E. Locci et al., "Test of a Lead-Plexipop Calorimeter Module Viewed by Wave Length Shifter Bars", Nucl. Instrum. Methods 164, (1979), S. 97-104,
S.W. Han et al., "Radiation Hardness Tests of Scintillating Tile/WLS Fiber Calorimeter Modules", Nucl. Instrum. Methods A365 (1995), S. 337-351.

Die in dem erfindungsgemäßen Photobioreaktor eingesetzten wellenlängenschieber umfassen vorzugsweise Fluoreszenzstoffe. Solche Fluoreszenzstoffe sind Stoffe, die nach Absorption von Licht wieder Licht abstrahlen, wobei die Energie für das abgestrahlte Licht im wesentlichen nicht dem Wärmeinhalt des Fluoreszenzstoffes entnommen wird, sondern aus der durch das absorbierte Licht zugeführten Anregungsenergie stammt.

Der Fluoreszenzstoff kann in einem Träger, wie einem organischen oder anorganischen Glas, enthalten sein.

Beispielsweise können organische Fluoreszenzstoffe in organischen Gläsern, wie Acrylglaspolycarbonat oder -polystyrol, enthalten sein. Ionen Seltener Erden, die ebenfalls als Fluoreszenzstoffe verwendet werden können, sind vorzugsweise in anorganischen Gläsern enthalten. Auch Lösungen der Fluoreszenzstoffe in transparenten Lösungsmitteln sind als Wellenlängenschieber einsetzbar.

Die Fluoreszenzstoffe besitzen die Eigenschaft, Licht zu absorbieren und, im allgemeinen nach sehr kurzer Zeit (häufig nur einige nsec), wieder zu emittieren. Wichtig ist, daß diese Reemission des Lichts bei manchen Substanzen nahezu verlustfrei geschieht, d.h. die Fluoreszenzquantenausbeute (Zahl der emittierten Photonen durch Zahl der absorbierten Photonen) ist >90%, oft nahe 100%. Ferner ist von Bedeutung, daß das Fluoreszenzspektrum gegenüber dem Absorptionsspektrum zu längeren Wellen hin verschoben ist, d.h. ein Farbstoff wandelt UV und violettes Licht in blaues Licht um, ein anderer blaues Licht in grünes Licht usw. Durch Kombination mehrerer Farbstoffe, entweder in derselben Platte oder z.B. durch Hintereinanderschichten verschieden eingefärbter Platten kann auch in einem Schritt ein größerer Wellenlängenbereich übersprungen werden, etwa direkt blaues in rotes Licht umgewandelt werden.

Von einem Wellenlängenschieber erwartet man hohe Fluoreszenzquantenausbeute, gute Löslichkeit im vorgesehenen Trägermaterial (um genügend hohe Lichtabsorption erreichen zu können), für die jeweilige Anwendung optimale Lage von Absorptionsund Emissionsspektrum und eine unter den vorgesehenen Einsatzbedingungen ausreichende Langzeitstabilität. Typische Beispiele für den Einsatz von Wellenlängenschiebern sind die Weißmacher in Waschmitteln, UV-absorbierende Farbstoffe, die blau emittieren, um das Vergilben der Wäsche zu überdecken, und die Farbstoffe, die in Szintillationszählern eingesetzt werden, um kurzwellige Cerenkov-Strahlung oder Szintillationsstrahlung in einen Wellenlängenbereich zu schieben, der für die spektrale Empfindlichkeit der eingesetzten Photodetektoren optimal ist.

Bevorzugte Beispiele für organische Fluoreszenzstoffe, die als Wellenlängenschieber in dem erfindungsgemäßen Photobioreaktor verwendet werden können, sind die folgenden Naphthalsäureimide und Perylenderivate: 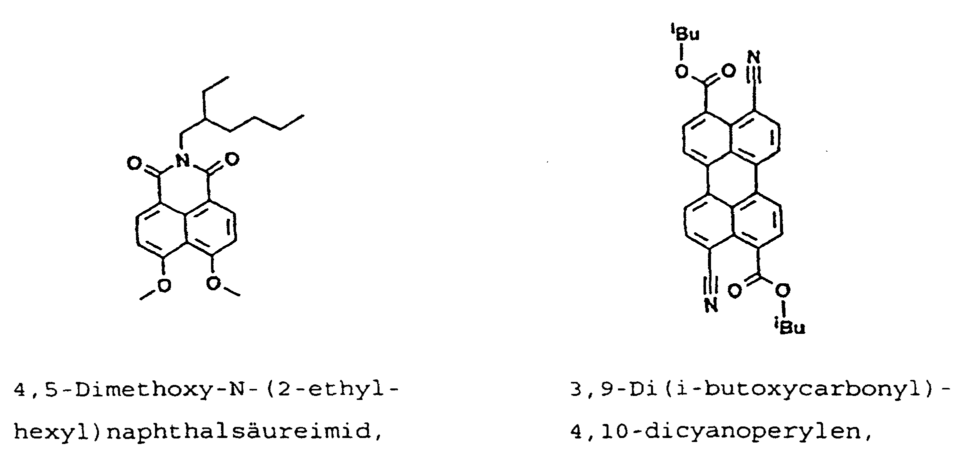 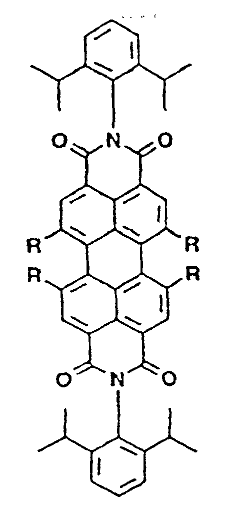 N,N'-Bis(2,6-diisopropylphenyl)perylen-3,4:9,10-tetracarbonsäurediimid bzw. N,N'-Bis(2,6-diisopropylphenyl)-1,6,7,12-tetraphenoxyperylen-3,4:9,10-tetracarbonsäurediimid.

Diese Fluoreszenzstoffe weisen alle eine sehr hohe Quantenausbeute auf. Die Maximalextinktionen liegen bei vielen 10.000 1/mol ·cm.

Die Fluoreszenzstoffe können in dem Trägermaterial in einer Konzentration von 10⁻⁷ bis 10⁻² mol/l vorliegen. Das Trägermaterial weist dann vorzugsweise eine Dicke von 0,1 bis 10 mm auf.

Durch die Verwendung eines Wellenlängenschiebers in Kombination mit der besseren räumlichen Verteilung des Lichts über den Reaktorquerschnitt durch Einstellung eines entsprechenden Oberflächen-/Volumen-Verhältnisses sowie durch die Erhöhung der Turbulenz zum Erreichen eines Flashing-Light-Effekts wird das Licht optimal in dem Reaktorraum des erfindungsgemäßen Photobioreaktors verteilt.

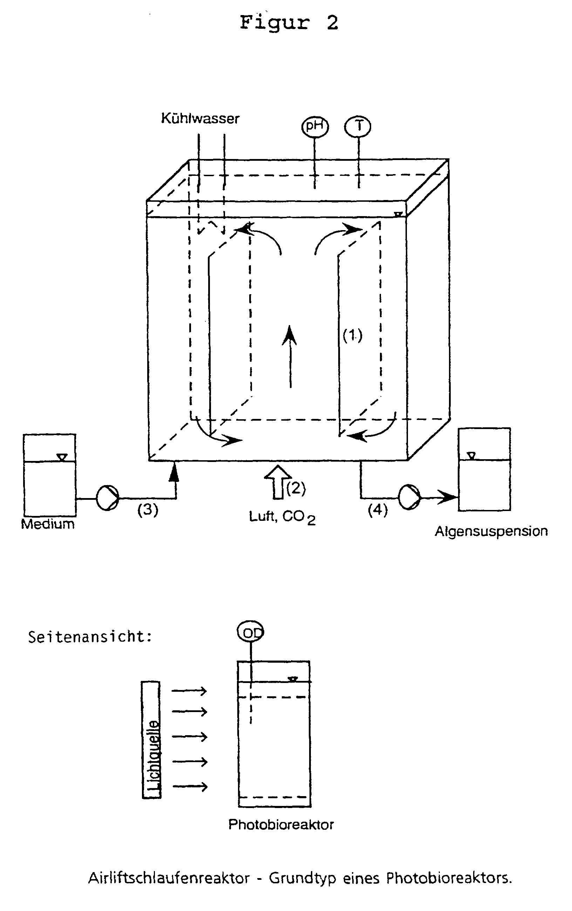

Die Figur 2 zeigt den Aufbau des erfindungsgemäßen Photobioreaktors nach dem Grundprinzip eines Airlift-Schlaufenreaktors. Der Reaktor weist einen rechteckigen Querschnitt und zwei Innenflächen (1) auf, die zu den Reaktorraumwänden parallel verlaufen, Einrichtungen zum Eindüsen von Luft an der Reaktorraumunterseite (2) sowie Einrichtungen zur Zuführung eines Mediums (3) sowie zur Entnahme der in dem Reaktorraum produzierten Biomasse (4).

Die Innenflächen dienen dabei als Leitrohr.

Als Material kann beispielsweise Plexiglas verwendet werden, das eine hohe Lichtdurchlässigkeit besitzt.

Die Durchmischung erfolgt durch Eindüsen von Luft an der Reaktorunterseite, so daß die Flüssigkeit nach oben strömt und seitlich wieder nach unten. Im hochturbulenten Kopfraum findet der Gasaustausch und die Temperierung statt. Die Temperierung im Kopfraum erspart eine zusätzliche Plexiglaswand, die bei einem Kühlmantel erforderlich wäre.

Durch den rechteckigen Grundaufbau und die geringe Reaktortiefe entsteht ein großes Oberflächen-/Volumen-Verhältnis und damit die Möglichkeit eines hohen Lichteintrages in den Reaktor. Der Reaktor hat keine unbeleuchteten Zonen, so daß den Zellen ständig ausreichend Licht zur Verfügung gestellt werden kann.

Der Betrieb als Airlift-Schlaufe sorgt für eine hohe Turbulenz bei geringen Scherkräften, die auf die Algenzellen wirken. Bei hoher Turbulenz und gleichzeitig hoher Strahlungsintensität kann der Flashing-Light-Effekt ausgenutzt werden, wonach die Zellen nicht ständig beleuchtet werden müssen. Die Turbulenz kann entweder durch die Begasungsrate erhöht werden, oder durch den Einbau von statischen Mischern (Strombrechern).

Bei einer Strömungsführung mit hohen Turbulenzen kommt es zusätzlich zur vertikalen Durchmischung zu einer horizontalen Durchmischung. Dadurch sind die phototrophen Mikroorganismen einer unterbrochenen Lichtversorgung unterworfen. Die Zykluszeit, die ein phototropher Mikroorganismus im Licht und dann im Dunkeln verbringt, sollte > 1 Hz betragen. Die Turbulenz bestimmt die Zeit, die die Mikroorganismen benötigen, um von der unbeleuchteten Zone wieder in die beleuchtete Zone zurück transportiert zu werden. Die Länge des Transportweges wird durch die Schichtdicke des Reaktors (Reaktortiefe) bestimmt.

Durch den Einbau von strömungsführenden Elementen wird die Turbulenz erhöht und damit kann man die Frequenz für den Transport der Mikroorganismen von der unbeleuchteten Zone wieder in die beleuchtete Zone verbessern.

Diese strömungsführenden Elemente könnten außerdem - ähnlich wie die Einbuchtungen der Vigreux-Kolonne - zusätzlich Licht in den Reaktor leiten.

Durch die gute Durchmischung und den ständigen Gasstrom im Reaktor ist ein guter CO₂-Eintrag und O₂-Austrag gewährleistet.

Die einfache Geometrie sowie die Anordnung der Entgasungszone in diesem System erlauben ein leichtes Up-scaling.

Statt eines Reaktors nach dem Prinzip der Airlift-Schlaufe kann der Reaktor auch als Wirbelzellenkolonne ausgestaltet sein.

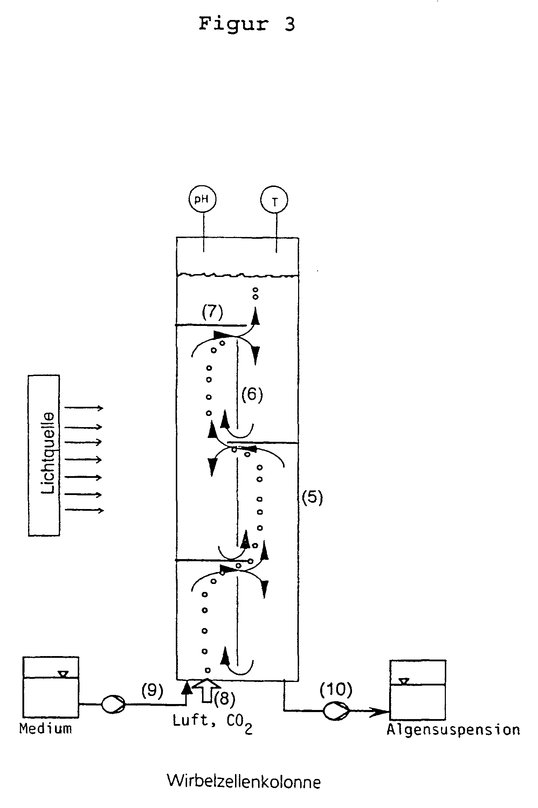

Eine solche Wirbelzellenkolonne ist in Figur 3 schematisch dargestellt. Hierin bezeichnet (5) einen Reaktorraum mit rechteckigem Querschnitt, der Innenwände (6,7) aufweist, die parallel (6) und im rechten Winkel (7) zu den Reaktorwänden (5) verlaufen.

Die Wirbelzellenkolonne weist weiterhin Einrichtungen zum Eindüsen von Luft an der Reaktorraumunterseite (8) sowie Einrichtungen zur Zuführung eines Mediums (9) sowie zur Entnahme der in dem Reaktorraum produzierten Biomasse (10) auf.

Die Innenflächen (6,7) dienen der Erzeugung einer turbulenten Strömung. Die Durchmischung erfolgt durch Eindüsen von Luft an der Reaktorunterseite, so daß die Flüssigkeit zunächst nach oben und anschließend seitlich wieder nach unten strömt. Durch die Querwände (7) werden die Gasblasen umgelenkt und führen zu einer Durchstömung und Wirbelbildung in der nächsten Zelle. Im Kopfraum findet der Gasaustausch statt. Durch die Wirbelbildung in den einzelnen Zellen findet eine intensive Durchmischung über den Reaktorquerschnitt statt.

Die voranstehend beschriebenen Airlift-Schlaufenreaktoren lassen sich auch in Serie nebeneinander anordnen, wobei sie fluidseitig nach dem Prinzip kommunizierender Röhren miteinander in Verbindung stehen.

Der erfindungsgemäße Bioreaktor kann auch so ausgestaltet sein, daß er mehrere Airlift-Schlaufenreaktoren aufweist, die als Wirbelzellenkolonne übereinander angeordnet sind.

Die Figur 4 schließlich zeigt den schematischen Aufbau eines Reaktorraums, der als Glasrohr ausgestaltet ist, in dessen Innenraum Glasfortsätze ragen.

Die Erfindung wird durch das nachfolgende Ausführungsbeispiel näher erläutert.

### Ausführungsbeispiel

Ein Rohrreaktor wurde durch die in Figur 4 dargestellten Einbuchtungen erweitert. Diese Einbuchtungen haben Doppelfunktion, indem sie sowohl die Reaktoroberfläche vergrößern als auch die Strömung beeinflussen. Vigreux-Kolonnen sind (ebenfalls aus Gründen der Oberflächenvergrößerung) nach diesem Prinzip aufgebaut und konnten direkt zu Vergleichstests herangezogen werden. Als Kontrolle wurde ein Rohrreaktor mit gleicher Geometrie ohne seitliche Einbuchtungen verwendet. Die Beleuchtung erfolgte mit 2 Halogenstrahlern, so daß 520 µE/(m²*s) Licht von einer Seite zur Verfügung stand. Die eingestrahlte Lichtintensität pro Volumen und Zeit war allerdings im Kontrollreaktor 10% niedriger als in der Vigreux-Kolonne.

**Tabelle:**

| Vergleich der Produktivität von Chlorella vulgaris in Reaktoren ohne und mit seitlichen Einbuchtungen (glattes Rohr und Vigreux-Kolonne) | | | | |
|---|---|---|---|---|
| | Trockengewicht TG(g/l) | Wachstumsrate µ(l/h) | Produktivität P(mg/l*h) | Änderung der Produktivität (%) |
| glattes Rohr | 2 | 0,016 | 32 | |
| | 4 | 0,0058 | 23,2 | |
| | 5 | 0,003 | 15 | |
| Vigreux-Kolonne Versuch 1 (V1) | 2 | 0,01 | 20 | -38 |
| | 4 | 0,0082 | 32,8 | 41 |
| | 5 | 0,006 | 30 | 100 |
| Vigreux-Kolonne Versuch 2 (V2) | 2 | 0,013 | 26 | -19 |
| | 4 | 0,007 | 28 | 21 |
| | 5 | 0,004 | 20 | 33 |

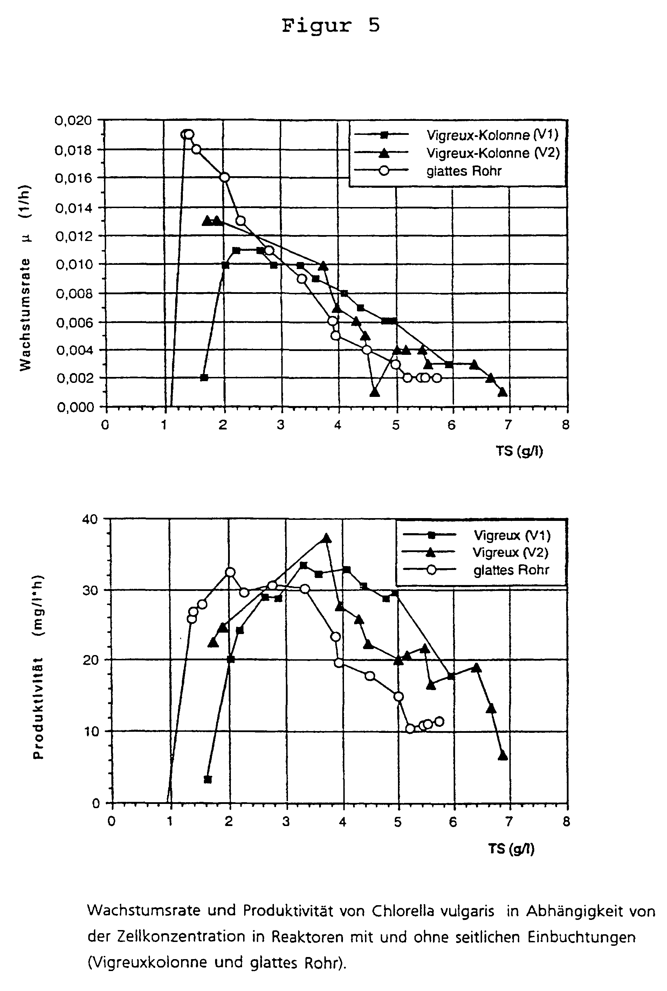

Der Direktvergleich ergab in der Vigreux-Kolonne eine um 20 bis 40% höhere Produktivität bei 4 g TG/l und 30 bis 100% höhere Produktivität bei 5 g TG/l (vergleiche Figur 5) im Vergleich zum Reaktor ohne seitliche Einbuchtungen. (Die unterschiedlichen Ergebnisse in V1 und V2 kommen durch eine unterschiedliche Voradaption der Algenzellen an das Licht zustande.)

Dies zeigt, daß durch eine Oberflächenvergrößerung, die gleichzeitig auch die Turbulenz erhöht, im Falle von Intensitäten oberhalb des Sättigungsbereiches, die Biomasseproduktivität deutlich gesteigert werden kann.

## Patentansprüche

1. Photobioreaktor, **dadurch gekennzeichnet, dass** er einen Reaktorraum mit Reaktorraumwänden aus lichtdurchlässigem Material aufweist, die ein Innenvolumen des Reaktorraums definieren, wobei der Reaktorraum eine vergrößerte Oberfläche aufweist, die größer ist als die geradflächige umhüllende Fläche des Innenvolumens des Reaktorraums.

2. Photobioreaktor nach Anspruch 1, **dadurch gekennzeichnet, daß** er weiterhin Einrichtungen für eine turbulente Strömungsführung aufweist.

3. Photobioreaktor nach Anspruch 1 oder 2, **dadurch gekennzeichnet, daß** er Elemente aufweist, die Licht von außen in den Reaktorraum leiten.

4. Photobioreaktor nach den Ansprüchen 1 bis 3, **dadurch gekennzeichnet, daß** die Umhüllende des Reaktorraumquerschnitts mäanderförmig oder sinusförmig ist.

5. Photobioreaktor nach den Ansprüchen 1 bis 3, **dadurch gekennzeichnet, daß** der Reaktorraum lichtdurchlässige Stege aufweist.

6. Photobioreaktor nach den Ansprüchen 1 bis 3, **dadurch gekennzeichnet, daß** der Reaktorraum als Glasrohr ausgestaltet ist, in dessen Innenraum Glasfortsätze ragen.

7. Photobioreaktor nach Anspruch 1 oder 2, **dadurch gekennzeichnet, daß** er als Airlift-Schlaufenreakcor oder in Form einer wirbelzellenkolonne ausgestaltet ist.

8. Photobioreaktor nach Anspruch 7, **dadurch gekennzeichnet, daß** der Reaktorraum einen rechteckigen Querschnitt, zu den Reaktorraumwänden parallel verlaufende Innenflächen (1), Einrichtungen zum Eindüsen von Luft an der Reaktorraumunterseite (2) sowie Einrichtungen zur Zuführung eines Mediums (3) sowie zur Entnahme der in dem Reaktor produzierten Biomasse (4) aufweist.

9. Photobioreaktor nach Anspruch 7 oder 8, **dadurch gekennzeichnet, daß** er mehrere Airlift-Schlaufenreaktoren aufweist, die in Serie nebeneinander angeordnet sind, wobei die einzelnen Airlift-Schlaufenreaktoren fluidseitig miteinander in Verbindung stehen.

10. Photobioreaktor nach Anspruch 7 oder 8, **dadurch gekennzeichnet, daß** er mehrere Airlift-Schlaufenreaktoren aufweist, die als Wirbelzellenkolonne übereinander angeordnet sind.

11. Photobioreaktor nach den Ansprüchen 1 bis 10, **dadurch gekennzeichnet, daß** er weiterhin einen Wellenlängenschieber aufweist.

## Claims

1. Photobioreactor, **characterized in that** it has a reactor chamber having reactor chamber walls made of light-transparent material defining an interior volume of the reactor chamber, wherein the reactor chamber has an increase in surface area greater than the planar enveloping surface of the interior volume of the reactor chamber.

2. Photobioreactor according to Claim 1, **characterized in that** it has, furthermore, devices for producing a turbulent flow conduction.

3. Photobioreactor according to Claim 1 or 2, **characterized in that** it has elements that conduct the light into the reactor chamber from the outside.

4. Photobioreactor according to Claims 1 to 3, **characterized in that** the envelope of the reactor cross section is me-ander-shaped or sinusoidal.

5. Photobioreactor according to Claims 1 to 3, **characterized in that** the reactor chamber has light-transparent webs.

6. Photobioreactor according to Claims 1 to 3, **characterized in that** the reactor chamber is designed as a glass tube into whose interior glass extensions project.

7. Photobioreactor according to Claim 1 or 2, **characterized in that** it is designed as an airlift loop reactor or in the form of a plate-fin column.

8. Photobioreactor according to Claim 7, **characterized in that** the reactor chamber has a rectangular cross section, internal surfaces (1) extending parallel to the reactor chamber walls, devices for injecting air at the lower side (2) of the reactor chamber, and devices for supplying a medium (3) and for removing biomass (4) produced in the reactor.

9. Photobioreactor according to Claim 7 or 8, **characterized in that** it has a plurality of airlift loop reactors that are disposed alongside one another in series, in which case the individual airlift loop reactors are connected to one another on the fluid side.

10. Photobioreactor according to Claim 7 or 8, **characterized in that** it has a plurality of airlift loop reactors that are disposed above one another as a plate-fin column.

11. Photobioreactor according to Claims 1 to 10, **characterized in that** it has, furthermore, a wavelength shifter.

## Revendications

1. Photobioréacteur, **caractérisé en ce qu'**il présente un compartiment de réacteur doté de parois du compartiment de réacteur en matériau transparent à la lumière, qui définissent un volume intérieur du compartiment de réacteur, le compartiment de réacteur présentant une surface élargie qui est plus grande que la surface enveloppante rectiligne du volume intérieur du compartiment du réacteur.

2. Photobioréacteur selon la revendication 1, **caractérisé en ce qu'**il présente en outre des dispositifs d'acheminement d'un flux à turbulences.

3. Photobioréacteur selon la revendication 1 ou 2, **caractérisé en ce qu'**il présente des éléments conduisant la lumière de l'extérieur dans le compartiment du réacteur.

4. Photobioréacteur selon les revendications 1 à 3, **caractérisé en ce que** la partie enveloppante de la section du compartiment du réacteur a une forme présentant des méandres ou une forme sinusoïdale.

5. Photobioréacteur selon les revendications 1 à 3, **caractérisé en ce que** le compartiment de réacteur présente des cloisonnements transparents à la lumière.

6. Photobioréacteur selon les revendications 1 à 3, **caractérisé en ce que** le compartiment de réacteur se présente sous la forme d'un tube de verre dans le compartiment intérieur duquel dépassent des prolongements en verre.

7. Photobioréacteur selon la revendication 1 ou 2, **caractérisé en ce qu'**il se présente sous la forme d'un réacteur à boucle à agitation pneumatique ou sous la forme d'une colonne à cellules à turbulences.

8. Photobioréacteur selon la revendication 7, **caractérisé en ce que** le compartiment de réacteur présente une section carrée, une surface intérieure (1) se prolongeant en parallèle aux parois du compartiment du réacteur, des dispositifs d'injection d'air sur le côté inférieur du compartiment du réacteur (2) ainsi que des dispositifs d'acheminement d'un milieu (3) et de prélèvement de la biomasse (4) produite dans le réacteur.

9. Photobioréacteur selon la revendication 7 ou 8, **caractérisé en ce qu'**il présente plusieurs réacteurs à boucle à agitation pneumatique qui sont disposés les uns à côté des autres en série, les réacteurs à boucle à agitation pneumatique individuels étant en communication de fluide.

10. Photobioréacteur selon la revendication 7 ou 8, **caractérisé en ce qu'**il présente plusieurs réacteurs à boucle à agitation pneumatique qui sont disposés les uns au-dessus des autres en une colonne de cellules à turbulences.

11. Photobioréacteur selon les revendications 1 à 10, **caractérisé en ce qu'**il présente en outre un élément de décalage de longueurs d'ondes.
